# EUROPEAN PATENT APPLICATION

(11) **EP 4 424 227 A1**
(43) Date of publication of application: **04.09.2024**
(21) Application number: 23159168.6
(22) Date of filing: 28.02.2023
(51) Int. Cl.: A61B 1/005, A61B 1/015, A61B 1/018

(54) **ENDOSCOPE HAVING A MEDIA TUBE**

(71) Applicant: Ambu A/S, 2750 Ballerup (DK)
(72) Inventor: CHRISTENSEN, Martin Johst, 2100 Copenhagen Ø (DK)
(74) Representative: AWA Denmark A/S

(57) **Abstract**

An endoscope (1) comprising an insertion cord (3), and a handle (2) including a control device (60). The insertion cord (3) comprises: a distal tip (10) having a discharge opening (13, 14); a bending section (20) having a proximal end and a distal end connected to the distal tip; a main tube (30) extending from the handle; at least one steering wire (25) attached to the control device (60) and running through the insertion cord (3) so that manipulation of the control device causes bending of the bending section; a working channel (31); and a first media tube (7) available for transporting a fluid from the handle to the discharge opening (13, 14) of the distal tip, the first media tube comprising a first portion (27) located in the main tube and a second portion (28) located in the bending section, the second portion comprising at least one corrugated section (17).

## Description

The present disclosure relates to an endoscope having a handle and an insertion cord, where the handle comprises a control device and the insertion cord comprises: a distal tip comprising a discharge opening; a bending section having a proximal end and a distal end connected to the distal tip; at least two steering wires attached to the control device and running through the insertion cord and connected to the distal end of the bending section so that manipulation of the control device causes bending of the bending section; a working channel. The disclosure further relates to a method for manufacturing an endoscope.

### Background

Flexible endoscopes for medical purposes are often provided with several tubes passing from a handle of the endoscope through an insertion cord, for insertion into body lumens, to the distal tip of the endoscope. These tubes serve different purposes. Usually, one tube is denoted a working channel and is applied for passing tools from the handle and to an area in front of the distal tip of the endoscope. Such tools may be for taking biopsies, or other surgical procedures. The working channel is often also applied for suctioning. Other tubes are typically applied for supply of fluids, i.e., liquid (e.g., water) or gas (e.g., carbon dioxide or atmospheric air). In this disclosure the term media tube is applied for the tubes intended for supply of fluids, but not intended for passage of tools or instruments. Often, the media tubes will have a smaller inner diameter than the inner diameter of the working channel.

Media tubes are often applied in endoscopes for the gastro-intestinal region, e.g., gastroscopes, colonoscopes, duodenoscopes. Here the need for cleaning of the distal tip window in front of the camera, and for cleaning of tissue surfaces before studying or performing a procedure, is more frequent. Also, introduction of carbon dioxide or atmospheric air is often used for insufflation to expand the lumen or cavity being investigated for improving the overview. Also, liquid or gaseous filling of a balloon, e.g., in connection with ultrasound imaging or temporary blockage of a body lumen, may require a separate media tube. At least in this last example, the liquid or gas may be transported in both directions in the media tube, i.e., from the handle towards the distal end and also from the distal end towards the handle. But in general, suctioning of fluids from an area in front of the distal tip of the endoscope, is typically done by application of the working channel.

Bending of the distal tip in a flexible endoscope is usually done by manually moving a bending lever or turning a control wheel, each arranged to pull steering wires connected to a bending section at the distal end of the insertion cord. It is important to keep the necessary force for performing the manual bending operation low in consideration of the operator of the endoscope, who may be doing the repetitive work of operating the endoscope for several hours a day.

Often, the endoscopes for gastro-intestinal procedures are four-way bending endoscopes. The outer diameter of their insertion cord may be in the range 9 - 12 mm, or larger. This large diameter, compared to bronchoscopes and cystoscopes, and the number of tubes passing through the bending section, may imply that a considerable force is needed to manually bend the bending section. It has been estimated that the tubes contribute to 25 - 60 % of the resistance to bending depending on the bending angle. Any reduction in this number will improve the ergonomic performance of the endoscope for the user.

### Summary

It is an object of the present disclosure to provide an endoscope where the necessary force for bending the media tubes passing through the bending section is reduced. The endoscope comprises at least one media tube as well as a working channel.

Thus, in one embodiment the present disclosure relates to an endoscope having a handle and an insertion cord, where the handle comprises a control device and the insertion cord comprises: a distal tip comprising a discharge opening; a bending section having a proximal end and a distal end connected to the distal tip; a main tube extending from the handle; at least one steering wire, attached to the control device and running through the insertion cord (and may be connected to the distal end of the bending section) so that manipulation of the control device causes bending of the bending section; a working channel, and a first media tube (different from the working channel) and being available for transporting fluid (e.g. liquid or gas) from the handle to the distal tip where it is deliverable through the discharge opening (e.g. one or more nozzles) to a space distal to the discharge opening of the distal tip, wherein the first media tube comprising a first portion located in the main tube and a second portion located in the bending section, the second portion comprising at least one corrugated section.

The advantage is that the addition of one or more corrugated sections to the media tube will reduce the force needed for bending the media tube. This reduces the force necessary for bending the bending section, and thereby less force is necessary for the manipulation of the control device.

For this disclosure one steering wire is counted as one passage from the control device to the distal end of the bending section. I.e. if the same unbroken steering wire continues from the distal end of the bending section and back to the control device, and one part is applied for bending for example to one side and the other is applied for bending to the opposite side, this is counted as two steering wires. Also, when distal wire ends are not connected to each other, but each attached to the distal end of the bending section or the tip part, this is also counted as two steering wires.

The discharge opening is an opening in the distal tip through which fluids from media tubes can be delivered to the space distal to the distal tip. The discharge opening may be a nozzle.

It may be preferred that the entirety of the media tube or media tubes is located inside the endoscope and extends from the distal tip to the connector at the end of the umbilical cord opposite to the endoscope handle. I.e. the media tube passes inside the endoscope through the bending section, the main tube, the handle, and the umbilical cord.

In a variation of the present embodiment, the insertion cord is further provided with a second media tube comprising a first portion located in the main tube and a second portion located in the bending section, wherein the second portion of the second media tube comprises at least one corrugated section. Often, an endoscope is provided with two or three media tubes, especially endoscopes for gastro-intestinal use, and it will provide the largest reduction in the force needed to bend the bending section when all media tubes are provided with corrugated sections, especially on their second portions passing through the bending section.

In a variation of the present embodiment the insertion cord defines a proximal to distal axial extending direction (defining longitudinal axial positions), where a corrugated section of the first media tube does not overlap with a corrugated section of the second media tube in any cross-section perpendicular to the axial extending direction. Since the corrugated sections may have a slightly larger outer diameter, it is an advantage to avoid overlap of corrugated sections to keep the outer diameter of the bending section as low as possible. This variation is at least designed for an unbent state of the bending section. But the dimensions of the corrugated and non-corrugated sections could also be designed so that there is no overlap of corrugated sections in a bent state of the bending section. This variation may preferably be related to second portions of the media tubes located in the bending section.

In a variation of the present embodiment at least the first media tube has at least two corrugated sections separated by a non-corrugated section, where the non-corrugated section overlaps with a corrugated section of the second media tube in the axial extending direction. This has the advantage of enabling more corrugated sections distributed in the bending section. Distributing corrugated sections non-overlappingly throughout the length of the bending section, may ensure that the shape of the fully bent bending section will not be affected by differences in the force necessary for bending the one or more media tube(s) when moving in a proximal to distal direction of the bending section.

In a further variation of the present embodiment, media tubes may also be provided with corrugated sections in the part of the insertion cord placed before the bending section. Also here, it will be preferred to arrange the corrugated sections so that they do not overlap.

In a variation of the present embodiment, the non-corrugated section of at least the first media tube is longer, in the axial extending direction, than the corrugated section. This has the advantage of avoiding overlap of the corrugated sections during bending, since the length of the corrugated section will expand on one side when bending while the length of the non-corrugated section will be constant, or at least substantially constant.

In a variation of the present embodiment, the working channel is not corrugated at the part placed in the bending section. Having a corrugated working channel especially in the bending section could increase the resistance to insertion of tools. Having a smooth inner surface without corrugations secures easy passage of tools and instruments also through a bending section bent to some extent.

In a variation of the present embodiment the endoscope is a four-way bending endoscope provided with four steering wires extending between the control device and the distal end of the bending section. The number of media tubes necessary in four way bending endoscopes for gastro-intestinal use is often higher, and the issue with a high necessary force for bending is therefore more obvious.

In a variation of the present embodiment the bending section of the endoscope includes segments, the segments including a proximal end segment at the proximal end, a distal end segment at the distal end, and intermediate segments arranged between the proximal end segment and the distal end segment. In an example of the present variation, the bending section of the endoscope is built from segments which are connected by hinges, and the segments and the hinges of the bending section are molded or fused together in one piece of polymer material. It has been found that such a bending section can be designed with a low resistance towards bending, and thereby supports the purpose of the corrugated sections of one or more media tubes.

In a second aspect the disclosure relates to a method for assembling an endoscope. This method comprises the steps: providing a first media tube having corrugated sections, a bending section and distal tip for an endoscope; attaching the first media tube to the distal tip; arranging the media tube in the bending section with at least one corrugated section inside the bending section; and attaching a proximal end of the media tube to a valve placed in an endoscope handle. In a variation of the method a second media tube with corrugated sections is provided and arranged similar to the first media tube, but arranging the corrugated sections such that a corrugated section of the first media tube does not overlap with a corrugated section of the second media tube in a cross-section perpendicular to a proximal to distal axial extending direction. This method has the advantage of ensuring that the corrugated sections of the media tubes are arranged in a pattern inside the bending section which takes up the least possible amount of space, and also ensures that the necessary force for bending the combined media tubes is the same or substantially the same through the length of the bending section.

In a third aspect the disclosure relates to a system comprising an endoscope as described above, a monitor and a control unit. The monitor and control unit may be separate parts or may be built together in one unit. Preferably, the endoscope is for single use, while the monitor and the control unit are reusable.

### Brief description of the figures

Fig. 1 shows an endoscope and a monitor with a control unit
Fig. 2 is a perspective view of a distal end of an endoscope without a bending cover.
Fig. 3 is a close-up view of intermediate segments of the bending section in fig. 2.
Fig. 4 is a cross-sectional view of a segment of a bending section including media tubes and steering wires passing through the bending section.
Fig. 5 is a view of a media tube with a corrugated section.
Fig. 6 is a view of a media tube with a corrugated section bent in the corrugated section.
Fig. 7 is examples of schematic depictions of parts of media tubes being partly corrugated.
Fig. 8 is a schematic illustration of three media tubes provided with corrugated sections placed in different longitudinal axial positions in the proximal to distal axial direction
Fig. 9 is a view of an example of a distal tip housing for an endoscope.
Fig. 10 shows an example of a camera module for an endoscope.
Fig. 11 shows an example of a control device, here in the form of a wire drum, and a wire pipe fastener.

### Detailed description

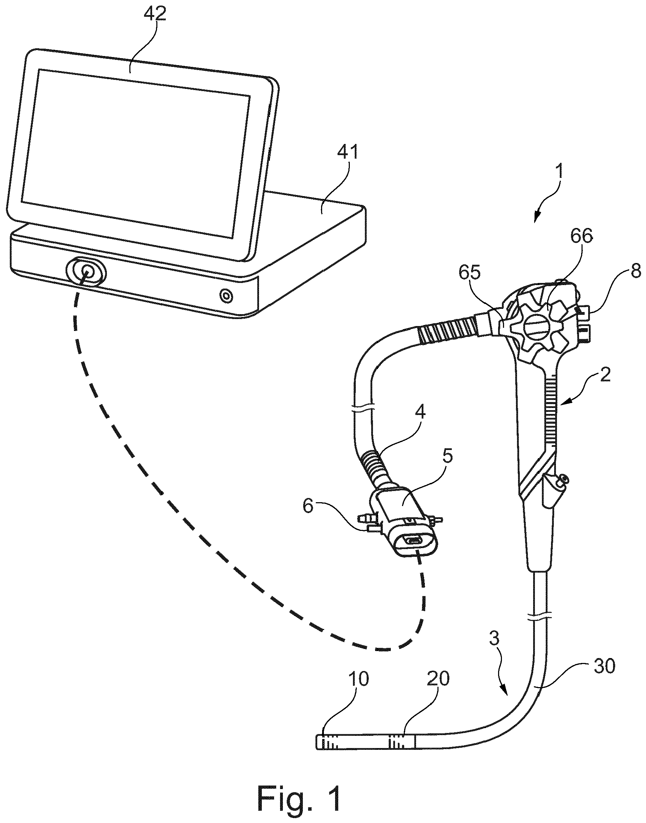

Fig. 1 illustrates an endoscope 1, which comprises a handle 2, an insertion cord 3 and an umbilical cord 4 with a connector 5 for connecting the endoscope 1 to a control unit 41 and a monitor 42. The insertion cord 3 is the part to be inserted into a body lumen during an endoscopic procedure. The insertion cord comprises three parts: a distal tip 10, a bending section 20 and a main tube 30.

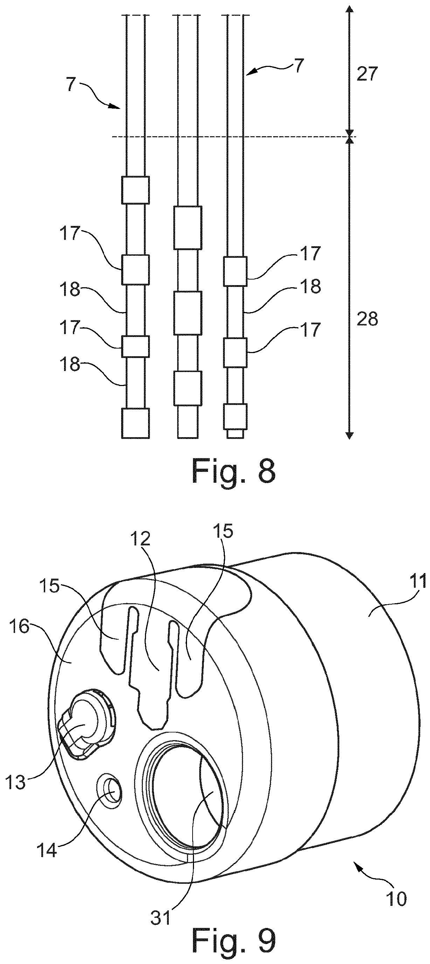

The distal tip 10 comprises a camera and light transmitters, e.g. in the form of one or more LEDs. Parts of the distal tip are often arranged in a distal tip housing 11 (as shown in fig. 9).

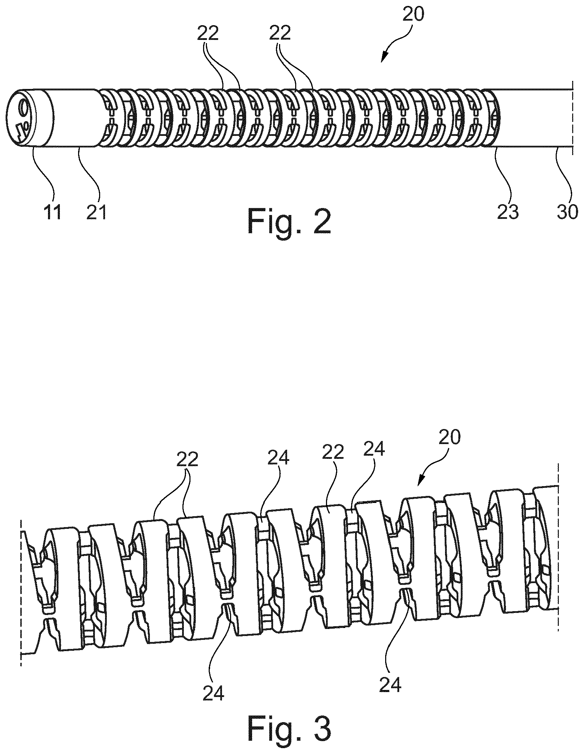

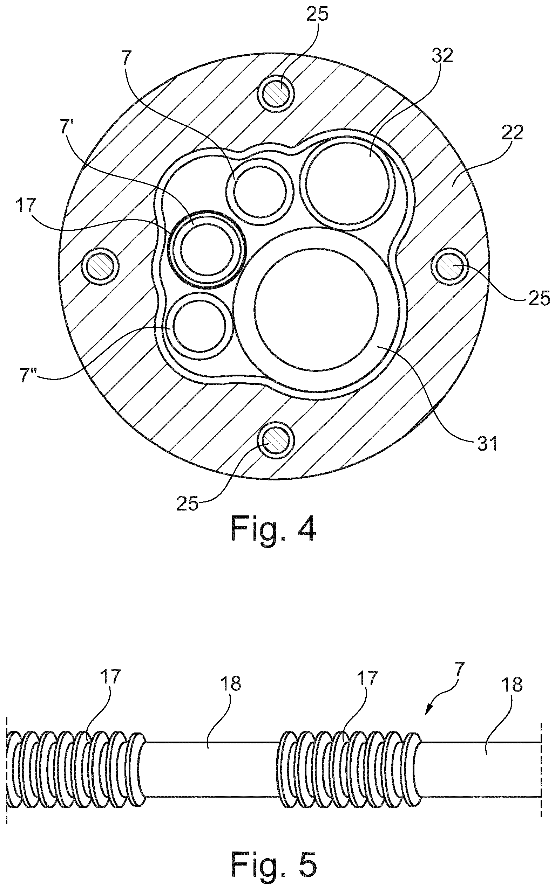

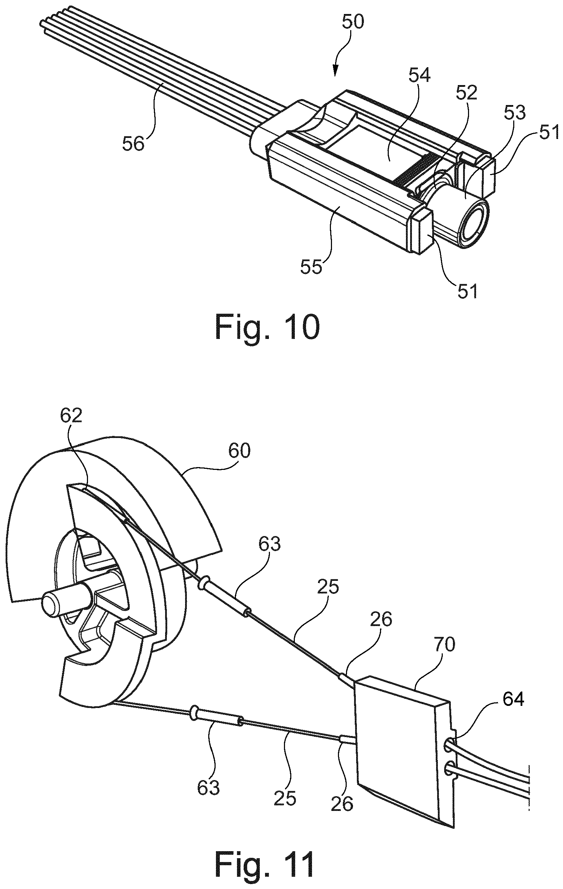

The bending section 20 may (as shown in figures 2 and 3) comprise a number of segments including a proximal end segment 23, a distal end segment 21, and intermediate segments 22. The segments are held together by hinges 24, so that the segments can be bent relative to each other by manipulation of the steering wires 25 (illustrated in fig. 4). Alternatively, the bending section 20 could be extruded in a relatively soft and resilient material, e.g. a foam-like material, with lumens for steering wires, electrical wires and the tubes passing through. Steering wires are connected in a fixed connection to the distal end, e.g. the distal end bending segment 21. I.e., the steering wire is preferably not movable in relation to the distal end bending segment. Between the handle 2 and the proximal end bending segment 23 the steering wire 25 is guided in a wire pipe 26 which is secured to a wire pipe fastener 70 placed in the handle 2 distal to the control device 60 (see fig. 11).

The main tube 30 for a four-way bending endoscope may be made from three parts: an inner metal coil, an intermediate metal braiding and an outer smooth polymer material. This construction of the main tube provides the desired torsional stiffness and resistance to kinking and is optimized to achieve the desired bending performance in the choice of materials and material dimensions.

The handle 2 in a four-way bending endoscope comprises two control wheels 65, 66 for manipulating steering wires 25 inside the handle 2. Turning one control wheel in one direction will pull one steering wire and make the bending section 20 bend in one direction. One control wheel 65, 66 will be configured to control the up-down direction, and the other control wheel will be configured to control the left-right direction. These directions are relative to a standing endoscope user having the insertion cord extending horizontally in the user's facial direction.

The control unit 41 and the monitor 42 may be separate parts, or they may be one combined part. The control unit will often comprise the electronic circuits for receiving and processing the image stream from the camera. The control unit 41 may be a platform where liquids or gas are supplied to relevant media tubes usually running inside the umbilical cord. The connector 5 is often provided with inlets 6 for liquids and gas to the media tubes. Bottles and containers (not shown) for these liquids or gas may be arranged close to, but on the exterior side of the control unit 41.

The media tubes 7 (see Figs. 4 to 8) may be made from a polymer, such as thermoplastic elastomers (TPE), thermoplastic polyurethanes (TPU), polyethylene, or polyether ether ketone (PEEK). But other polymers may also be applied. The media tubes passing through the insertion cord may for example have inner diameters in the range 0.8 - 2.0 mm, more often in the range 1.0 - 1.5 mm. The wall thickness of the tubes in the sections where they are not corrugated may be in the range 0.1 - 0.4 mm, or more often in the range 0.15 - 0.3 mm.

Often the inner diameter of the part of the media tubes 7 passing through the umbilical cord 4 from the inlets 6 to the valves 8 in the handle will be slightly larger than these ranges to keep the total pressure drop for the media transport as low as possible. For example, the inner diameter of the media tubes passing the umbilical cord may be in the range 2-3 mm. In the handle the media tubes are connected to valves 8 controlling the flow of fluid. The valves are connected to the media tubes passing through the insertion cord 3 to the distal tip 10. Such valves are usually manually operated by the endoscope user, who can apply the flow of fluid as needed to perform a procedure.

Fig. 2 shows a distal end of the insertion cord 3 of an endoscope, where the bending cover (not shown) typically covering the bending section 20 part and often makes this part watertight, has been removed for illustration purposes. The bending section is attached to the main tube 30 in the proximal end, and the distal tip housing 11 in the distal end. In this example the bending section is molded in one piece and comprises segments 21, 22, 23 connected by hinges 24. This is seen more clearly in fig. 3 showing a close-up view of intermediate segments 22 of the bending section. Other designs of the bending section are also possible.

Fig. 4 shows a cross-sectional view of one intermediate segment 22 of the bending section in fig. 3, including media tubes 7, working channel 31 and a sleeve with electrical wires 32 passing through the bending section. This cross-sectional view shows the second portion 28 of the media tubes (see also fig. 8), i.e., the portion of the media tubes 7 placed inside the bending section 20.

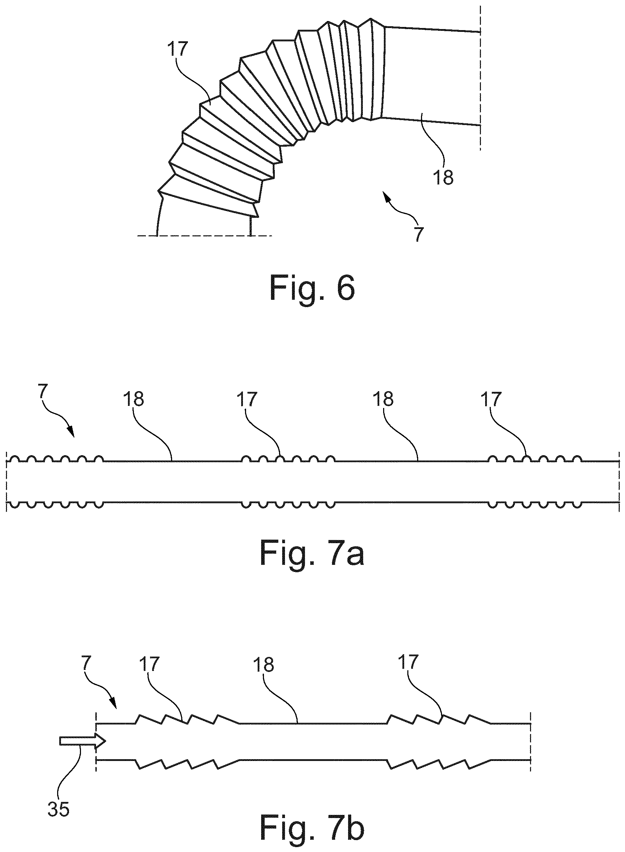

Fig. 5 - 7 shows different examples of parts of media tubes 7 having corrugated sections 17 and non-corrugated sections 18 or being partly corrugated. Fig. 6 shows an example of a part of a media tube with a corrugated section being bent in the corrugated section 17. Fig. 7a illustrates schematically, in a sectional view in a plane coincident with the proximal to distal center axis, corrugated sections 17 with intermediate non-corrugated sections, where the corrugations are made from half circles separated by straight parts. Fig. 7b is similar to fig. 7a, but here each corrugation has a different wall for the proximal and the distal part of the corrugation. The arrow 35 shows the proximal to distal direction, which is also most often the flow direction of the fluid. A corrugation wall with a steep angle at the proximal side and a more sloped angle at the distal side (as shown in fig. 7b), may cause less opposition to flow for the same corrugation length.

Fig. 8 shows an illustrative example of three media tubes provided with corrugated sections placed in different longitudinal axial positions. The relative longitudinal axial positions of the corrugated sections between these three media tubes may be kept constant as shown in fig. 8 during assembly of the endoscope and insertion of the media tubes into the bending section. As illustrated, the media tubes comprise a first portion (27) located in the main tube and a second portion (28) located in the bending section. As described herein, the corrugated sections are preferably located in the second portion (28).

The corrugations in the corrugated sections of the media tubes 7 may have different shapes. The shape may follow a sinusoidal waveform, a triangular waveform, a square waveform, a wave form made up of half circles or any other waveform or combination of waveforms. The waveform can also be characterized by a frequency, e.g. as number of cycles per millimeter, and by an amplitude, e.g. half the distance from an inward facing peak to an outward facing peak measured in mm. The amplitude is measured along a radial direction from the center axis of the media tube. For a media tube with an outer diameter in the range 1.1 - 2.6 mm examples of the frequency could be in the range 3 - 15 cycles/mm. Corresponding examples of the amplitude could be in the range 0.03 - 0.25 mm. Both the frequency and the amplitude will be related to the dimensions of the media tube.

The frequency and the amplitude of the corrugations in the corrugated sections 17 of the one or more media tubes 7 will influence and reduce the force needed to bend the one or more tubes when the tubes are seen as an isolated component. This reduction in force for bending the media tubes will also result in a reduced force needed to bend the bending section 20 in the assembled endoscope. The corrugated sections of the media tubes in the bending section will also reduce the risk of the media tubes kinking, and thereby blocking or reducing the flow of water or gas, especially when the bending section 20 is bent at maximum bending angle.

A major part of the force for bending a bending section 20 having media tubes 7 without corrugations is related to the necessary displacement of the media tubes inside the bending section and the main tube 30. This displacement takes place in the longitudinal axial direction of the insertion cord 3 and can be explained by introducing the terms a concave side of any bent tube and an opposite convex side of the bent tube. The concave side is the side where the tube curves inward, and the surface of the tube is compressed. The convex side is where the tube curves outward, and the surface of the tube are extended.

When a media tube is placed closest to the convex side of a bent bending section, the full passage length from the distal to the proximal end of the bending section will be extended for this media tube. The part of the media tube inside the bending section will therefore cause a drag or pull in the part of the media tube placed in the main tube. This drag will tend to displace the media tube in the main tube towards the bending section. When a media tube is placed closest to the concave side of a bent bending section, the full passage length from the distal to the proximal end of the bending section will be shorter, and this media tube will be compressed. The part of the media tube inside the bending section will therefore cause a pushing force on the part of the media tube placed in the main tube. This push will tend to displace the media tube in the main tube away from the bending section.

The necessary force for these displacements of the media tubes during bending will add some force to the total force needed to bend the bending section. This can only be avoided for a media tube arranged such that the center axis of the media tube coincides with the longitudinal center axis of the bending section. In practice, this is however seldom possible.

However, when adding at least one corrugated section to a media tube inside the bending section the displacement of the media tube in the main tube can be avoided as the pulling and pushing forces in the media tube when bending the bending section can be absorbed in the corrugated section which is compressed and extended instead. The forces needed for compressing and extending the corrugated sections will be significantly smaller than the necessary forces for displacing the media tube inside the main tube.

When the corrugated sections are formed as part of an extruding process, it is possible to obtain the same material thickness of the tube wall as in the non-corrugated parts of the tube. If the corrugated sections are formed in an existing tube, e.g. by a heat forming process, the wall thickness of the noon-corrugated tube should preferably be large enough to provide the extra material needed in the corrugated sections, to secure that the wall thickness in the corrugated section will be large enough to provide the needed mechanical properties. So, in this case a larger initial wall thickness may be needed. The initial wall thickness could for example be 5 - 15% larger as compared to the wall thickness selected for a corresponding media tube without corrugated sections.

Often, the inner diameter of the media tube is designed to obtain a given flow of fluid at a given pressure. Therefore, it will be preferred to provide the corrugated sections such that the inner diameter is not reduced in these sections, and thereby not reducing the flow at a given pressure difference. This means that the extra space taken up by the corrugations will be added to the overall outer diameter of the media tubes in the corrugated sections. The overall outer diameter of the corrugated sections may be increased in the range from 3 % up to 25 %, or preferably in the range from 5 % up to 20 %, and more preferably in the range 5 % up to 15 %.

In order to keep the outer diameter of the bending section as low as possible, it is preferred that when more than one media tube is used, the corrugated sections of the media tubes in the bending section do not overlap in the proximal to distal axial extending direction defined by the insertion cord. This mean that in any cross-section through the bending section perpendicular to this longitudinal axial extending direction, there should preferably not be more than one media tube with a corrugated section. Alternatively, this limitation may only apply to two media tubes placed closely together, and potentially abutting each other during bending of the bending section. Referring to figure 4 this can be exemplified in that media tube 7' cannot have a corrugated section overlapping with neither a corrugated section of media tube 7 nor of media tube 7". However, media tube 7 and media tube 7" may have an overlapping corrugated section, provided that the design of the bending section is such that these will not be able to abut each other. In fig. 4, it is shown that only media tube 7' has a corrugated section 17 at this specific cross section.

When two or more abutting media tubes are arranged in the bending section each with alternately corrugated 17 sections and non-corrugated sections 18, and where the corrugated sections do not overlap as described above, it may be preferred that the non-corrugated sections 18 are longer than the corrugated sections 17. The reason is that when a first media tube 7 is bent the corrugated sections 17 will be extended in length on the convex side, whereas the non-corrugated sections of an abutting second media tube will keep a constant or substantially constant length also on the abutting concave side of the second media tube. So, to avoid overlap of corrugated sections, the non-corrugated section 18 of the media tubes 7 should be longer than the corrugated sections 17. How much longer the non-corrugated sections should be will depend on the specific dimension of the media tubes, the length and number of corrugated sections and of the length, maximum bending angle and bending radius of the bending section.

Fig. 9 shows an example of a distal tip 10 of an endoscope. The distal comprises a distal tip housing 11 inside which a camera module 50 is arranged. The distal tip housing 11 comprises on a distal front surface 16 a transparent camera window 12 and windows 15 in front of a light source such as an LED 51, and further a discharge opening or nozzle 13 for rinsing the camera window 12 with water irrigation and for insufflation of gas, e.g. for inflating a cavity slightly. The distal front surface 16 is in this example provided with a further discharge opening forming a waterjet nozzle 14, for cleaning e.g. a tissue surface to be able to see the surface. Also, the distal end of the working channel 31 is seen in fig. 9.

The nozzle 13 for both rinsing water and gas insufflation is connected to two media tubes 7 one for water and one for gas. The nozzle 13 could also be connected to only one media tube branching out to two media tubes via a Y-connector (not shown). Such branching should be as close to the nozzle 13 as possible to minimize the amount of gas or water in the one media tube which has to be emptied when shifting to the other fluid. The waterjet nozzle 14 is connected to one media tube supplying water. The supply of water and gas to the nozzles 13, 14 is controlled by valves 8 at the handle 2.

Fig. 10 shows an example of a camera module 50 typically arranged in a distal tip housing 11. The camera module comprises an image sensor 52, LEDs 51 as light sources, a lens stack 53, a flexible printed circuit board 54 connecting the image sensor 52 and LEDs 51 to the wires 56 which are connected to a circuit board in the handle. A camera module frame 55 made of a polymer material typically supports and keeps the other parts in the correct position.

Fig. 11 shows an example of a control device 60 configured for bending the bending section by pulling the steering wires 25. The steering wires 25 are moved by rotation of a wire drum 61, which is a circular arc surface or a curved surface of the control device supporting the steering wire. In the example one end of the steering wire has been drawn through a small lug 62 and bent back and fastened to itself by a crimp 63. This system is described further in US 2021/0137355 A1, which is incorporated herein by reference in its entirety, and works for a two-way bending endoscope. For a four-way bending endoscope two independently rotatable wire drums are typically applied. This is indicated in fig. 1 by the two different control wheels 65, 66, where a control device 60 is manipulated to bend the bending section 20 through rotation of a control wheel 65, 66.

Fig. 11 further shows the wire pipe fastener 70 into which the wire pipes 26 are often fixated e.g. by gluing in a glue passage 64. The wire pipe fastener is secured inside the handle, often directly to a handle shell. The distal end of the wire pipes 26 are usually fixated and terminated at the proximal end of the bending section 20.

### List of references

| | |
|---|---|
| 1 endoscope | 27 first portion of media tube |
| 2 handle | 28 second portion of media tube |
| 3 insertion cord | 30 main tube |
| 4 umbilical cord | 31 working channel |
| 5 connector | 32 sleeve with electrical wires |
| 6 inlets to media tubes | 35 flow direction |
| 7 media tube | 41 control unit |
| 8 valves | 42 monitor |
| 10 distal tip | 50 camera module |
| 11 distal tip housing | 51 LED |
| 12 camera window | 52 image sensor |
| 13 nozzle for rinsing and insufflation | 53 lens stack |
| 14 waterjet nozzle | 54 flexible printed circuit board |
| 15 window in front of light source | 55 camera module frame |
| 16 distal front surface of distal tip | 56 wires |
| 17 corrugated section | 60 control device |
| 18 non-corrugated section | 61 wire drum |
| 20 bending section | 62 lug for steering wire |
| 21 distal end segment | 63 crimp |
| 22 intermediate segment | 64 glue passage |
| 23 proximal end segment | 65 control wheel |
| 24 hinges | 66 control wheel |
| 25 steering wire | 70 wire pipe fastener |
| 26 wire pipe | |

## Claims

1. An endoscope comprising:
a handle comprising a control device; and
an insertion cord including:
- a distal tip comprising a discharge opening;
- a bending section having a distal end connected to the distal tip;
- a main tube extending from the handle;
- at least one steering wire attached to the control device and running through the insertion cord so that manipulation of the control device causes bending of the bending section;
- a working channel; and
- a first media tube available for transporting a fluid from the handle to the discharge opening of the distal tip, the first media tube comprising a first portion located in the main tube and a second portion located in the bending section, the second portion comprising at least one corrugated section.

2. The endoscope according to claim 1, wherein the insertion cord further comprises a second media tube comprising a first portion located in the main tube and a second portion located in the bending section, the second portion comprising at least one corrugated section.

3. The endoscope according to claim 2, wherein the insertion cord defines a proximal to distal axial extending direction, where a corrugated section of the first media tube does not overlap with a corrugated section of the second media tube in any cross-section perpendicular to the axial extending direction.

4. The endoscope according to claim 3, wherein at least the first media tube has at least two corrugated sections separated by a non-corrugated section, wherein the non-corrugated section overlaps with a corrugated section of the second media tube in the axial extending direction.

5. The endoscope according to claim 4, wherein the non-corrugated section is longer, in the axial extending direction, than the corrugated section.

6. The endoscope according to claim 1, wherein the working channel is not corrugated at the part placed in the bending section.

7. The endoscope according to any one of the previous claims, wherein the endoscope is a four-way bending endoscope provided with four steering wires extending between the control device and the distal end of the bending section.

8. The endoscope according to any one of the previous claims, wherein the bending section includes segments, the segments including a proximal end segment at the proximal end, a distal end segment at the distal end, and intermediate segments arranged between the proximal end segment and the distal end segment.

9. The endoscope according to claim 8, wherein the segments are connected by hinges, and the segments and the hinges of the bending section are molded or fused together in one piece of polymer material.

10. A method for assembling an endoscope, comprising the steps of:
- providing a first media tube having corrugated sections, a bending section and distal tip for an endoscope,
- attaching the first media tube to the distal tip,
- arranging the media tube in the bending section with at least one corrugated section inside the bending section,
- attaching a proximal end of the media tube to a valve placed in an endoscope handle.

11. The method according to claim 10, wherein a second media tube with corrugated sections is provided and arranged similar to the first media tube but arranging the corrugated sections such that a corrugated section of the first media tube does not overlap with a corrugated section of the second media tube in a cross-section perpendicular to a proximal to distal axial extending direction.

12. A system comprising an endoscope according to any one of claims 1-9, a monitor and a control unit.
